# FASCICULE DE BREVET EUROPEEN

(11) **EP 0 523 154 B1**
(45) Date de publication et mention de la délivrance du brevet: **11.01.1995**
(21) Numéro de dépôt: 91907726.3
(22) Date de dépôt: 05.04.1991
(51) Int. Cl.: F16M 11/04

(54) **BRAS A EXTREMITE TRANSLATABLE ET APPAREIL DE TRAITEMENT THERAPEUTIQUE, EN COMPORTANT APPLICATION**
STATIVARM MIT VERSCHIEBBAREM ENDE SOWIE APPARAT MIT DEMSELBEN FÜR THERAPEUTISCHE BEHANDLUNG
ARM WITH TRANSLATABLE END AND APPARATUS FOR THERAPEUTIC TREATMENT, COMPRISING APPLICATION THEREOF

(30) Priorité: 06.04.1990 FR 9004440
(43) Date de publication de la demande: 20.01.1993
(73) Titulaire: TECHNOMED MEDICAL SYSTEMS, 69120 Vaulx-en-Velin (FR)
(72) Inventeur: LACOSTE, François, F-69006 Lyon (FR); DEVONEC, Marian, F-01700 Miribel (FR); CATHAUD, Muriel, F-69780 Vénissieux (FR)
(74) Mandataire: Portal, Gérard
(86) Numéro de dépôt international: FR9100277
(87) Numéro de publication internationale: WO9115708

(56) Documents cités:
- EP-A- 0 248 758
- EP-A- 0 368 161
- DE-A- 1 901 180
- FR-A- 2 388 198
- FR-A- 2 502 485
- FR-A- 2 598 073
- NL-A- 6 403 232
- US-A- 2 202 195
- US-A- 3 638 973
- Ultrasound in Med. & Biol., volume 5, No. 2, 1979, Pergamon Press, (GB), W.B. Taylor et al.: "A high-resolution transrectal ultrasonographic system", pages 129-138 see figure 3

## Description

La présente invention concerne essentiellement un bras à extrémité translatable ainsi qu'un appareil de traitement thérapeutique, en comportant application.

Il est connu par le document FR-A-2 502 485 un appareil d'exploration à sonde pour le diagnostic par ultrasons, comprenant un bras essentiellement rigide ayant deux extrémités, une première extrémité articulée relativement à un bâti support par au moins un axe d'articulation, et de préférence par au moins deux axes d'articulation, et une deuxième extrémité comprenant deux axes d'articulation perpendiculaires supportant un deuxième bras a l'extrémité libre duquel il est prévu un élément support d'une sonde d'exploration.

La sonde d'exploration montée sur ce bras ne peut pas être translatée à l'extrémité du bras, ce qui constitue un inconvénient dans le cadre de certaines applications thérapeutiques, en particulier dans le cas d'exploration médicale, notamment par la voie endorectale.

On connaît aussi par le document FR-A-2 598 073 du même déposant un dispositif de positionnement dans l'espace d'une sonde d'exploration occupant un volume limité. Le dispositif de positionnement est particulièrement adapté pour le repérage de lithiase dans le cadre de la lithotritie.

Cependant, ce dispositif ne prévoit pas la possibilité de translater la sonde d'exploration, ce qui limite son application dans le cadre thérapeutique, notamment pour l'exploration par voie endorectale.

La présente invention a pour but de résoudre le nouveau problème technique consistant en la fourniture d'un dispositif de positionnement dans l'espace d'un instrument, permettant un mouvement de translation de cet instrument.

La présente invention a également pour but de résoudre le nouveau problème technique consistant en la fourniture d'un dispositif de positionnement dans l'espace d'un instrument, offrant un maximum de liberté de positionnement dans l'espace de cet instrument, d'une conception simple, de fabrication aisée, et relativement peu coûteuse à l'échelle industrielle.

La présente invention a encore pour but de résoudre le nouveau problème technique consistant en la fourniture d'un dispositif de positionnement dans l'espace d'un instrument comprenant une sonde d'exploration, notamment permettant de réaliser des explorations échographiques, en particulier par la voie endorectale d'une manière extrêmement simple, efficace et fiable.

Avec le dispositif du positionnement dans l'espace selon l'invention, on peut également positionner une sonde d'exploration quelconque, en particulier une sonde d'exploration radiographique, notamment par rayons X.

Ces problèmes techniques sont résolus pour la première fois simultanément par la présente invention.

Ainsi, selon un premier aspect, la présente invention fournit un dispositif de positionnement dans l'espace d'un instrument, notamment comprenant une sonde d'exploration par visualisation, comprenant au moins un bras essentiellement rigide ayant une première extrémité et une deuxième extrémité, la première extrémité étant articulée relativement à un bâti support par au moins un axe d'articulation, et ledit instrument étant monté sur un élément support relié à la deuxième extrémité du bras par au moins un axe d'articulation, caractérisé en ce que ledit instrument comprend une sonde endocavitaire ou une sonde d'exploration notamment d'exploration par visualisation, en particulier une sonde échographique, de préférence de type rectal, permettant une exploration endorectale, et ledit élément support de l'instrument comprend un dispositif de translation permettant de réaliser une translation de l'instrument comprenant ladite sonde endocavitaire ou d'exploration, par rapport à la deuxième extrémité du bras, en permettant de réaliser une opération, notamment une exploration, pour chacune des positions de translation.

Selon un mode de réalisation avantageux de l'invention, le dispositif de positionnement comprend deux bras articulés entre eux par au moins une articulation, ainsi qu'à chacune de ses extrémités libres, soit au total au moins trois articulations.

Selon une autre variante de l'invention, ledit dispositif de positionnement comprend des moyens de blocage simultané des articulations.

Selon encore une autre variante de réalisation, ledit dispositif de positionnement, dans lequel les articulations comprennent des éléments mobiles et des éléments fixes, est caractérisé en ce que les moyens de blocage réalisent un blocage provisoire par déplacement relatif des éléments mobiles et fixes des articulations et sont en particulier de type pneumatique ou hydraulique.

Selon un autre mode de réalisation, ledit dispositif de positionnement est caractérisé en ce que les moyens de blocage comprennent au moins un passage interne continu, permettant le passage à l'intérieur du dispositif de positionnement d'un fluide pneumatique ou hydraulique de mise sous pression, lors de l'activation du blocage, les bras étant alors formés par des tubes.

Selon un mode de réalisation avantageux de l'invention, le dispositif de translation précité comprend un élément fixe solidaire de l'articulation de la deuxième extrémité du bras par un organe de liaison, et un élément mobile déplaçable en translation relativement à l'élément fixe, supportant ledit instrument ; ou inversement.

Selon un autre mode de réalisation particulièrement avantageux de l'invention, l'élément mobile du dispositif de translation comprend des moyens de mise en rotation de l'instrument, permettant un mouvement de rotation de l'instrument.

Selon une caractéristique particulière de l'invention, les moyens de mise en rotation précités comprennent un organe de débrayage permettant une mise en rotation manuel le de l'instrument.

Selon une autre caractéristique avantageuse du dispositif selon l'invention, l'élément mobile précité a une forme en L dont une branche se déplace en translation relativement à l'élément fixe du dispositif de translation, et dont l'autre branche supporte l'instrument, avantageusement de manière rotative.

Selon un mode de réalisation préféré du dispositif de positionnement selon l'invention, au moins une articulation, de préférence toutes les articulations, sont des articulations de type rotule.

Selon un mode de réalisation avantageux, l'articulation de type rotule à la deuxième extrémité du bras précitée est de type inversé, de sorte que le bras est solidarisé à sa première extrémité et à sa deuxième extrémité à la partie interne, habituellement mobile, de l'articulation de type rotule.

Selon une autre caractéristique particulièrement préférée de l'invention, au moins certains des éléments fixes des articulations type rotule ont leur axe de symétrie formant un angle d'inclinaison aigu relativement à l'axe général du bras. Cet angle d'inclinaison est avantageusement compris entre 20 et 65°, de préférence entre 35 et 60°, encore de préférence d'environ 55°.

Selon une autre caractéristique avantageuse de l'invention, l'angle d'inclinaison entre l'élément dit fixe de la dernière articulation de type rotule à la deuxième extrémité du bras et l'organe de liaison à l'élément fixe du dispositif de translation est différent de celui des autres articulations de type rotule.

Selon un deuxième aspect, la présente invention concerne aussi un appareil de traitement thérapeutique, caractérisé en ce qu'il comprend un dispositif de positionnement dans l'espace d'un instrument, comprenant une sonde endocavitaire ou une sonde d'exploration, tel que précédemment défini.

Selon un mode de réalisation avantageux de cet appareil de traitement thérapeutique, la sonde précitée est une sonde endorectale, permettant une exploration endorectale, avantageusement pour l'exploration de la prostate par voie endorectale. Cette sonde peut également être une sonde d'exploration par voie ultrasonique ou par voie radiographique, notamment par rayons X.

Selon un autre mode de réalisation, l'appareil est un appareil de traitement par hyperthermie, notamment de la prostate.

On comprend ainsi que l'on obtient tous les avantages techniques déterminants précédemment mentionnés, en permettant ainsi notamment de réaliser une translation d'un instrument supporté par le dispositif de positionnement, tout en maintenant des degrés de liberté maximum pour permettre un positionnement de l'instrument dans n'importe quelle position dans l'espace.

D'autres buts, caractéristiques et avantages de l'invention apparaîtront clairement à la lumière de la description explicative qui va suivre faite en référence aux figures annexées représentant un mode de réalisation actuellement préféré du dispositif de positionnement selon l'invention, donné simplement à titre d'illustration et qui ne saurait donc en aucune façon limiter la portée de l'invention.

Dans les dessins :
- la figure 1 est une vue schématique en perspective des parties essentielles du dispositif de positionnement dans l'espace d'un instrument, tel qu'une sonde d'exploration, en particulier de type échographique, selon un mode de réalisation actuellement préféré de l'invention, pouvant être utilisée dans un appareil de traitement thérapeutique, en particulier pour un traitement par hyperthermie, notamment de la prostate ;
- la figure 2 est une vue en coupe partielle transversale longitudinale montrant les articulations de type rotule du dispositif de positionnement représenté à la figure 1, le dispositif de translation étant enlevé ;
- la figure 3 est une vue de dessus représentant le dispositif de translation dans deux positions de translation différentes montrant le déplacement par translation de l'instrument supporté par le dispositif ;
- la figure 4 représente une vue en coupe de détails agrandis d'une articulation de type rotule selon l'invention ;
- la figure 5 représente une vue en coupe selon la ligne de trace V-V de la figure 1 ; et
- la figure 6 représente une vue en coupe selon la ligne de trace VI-VI de la figure 1.

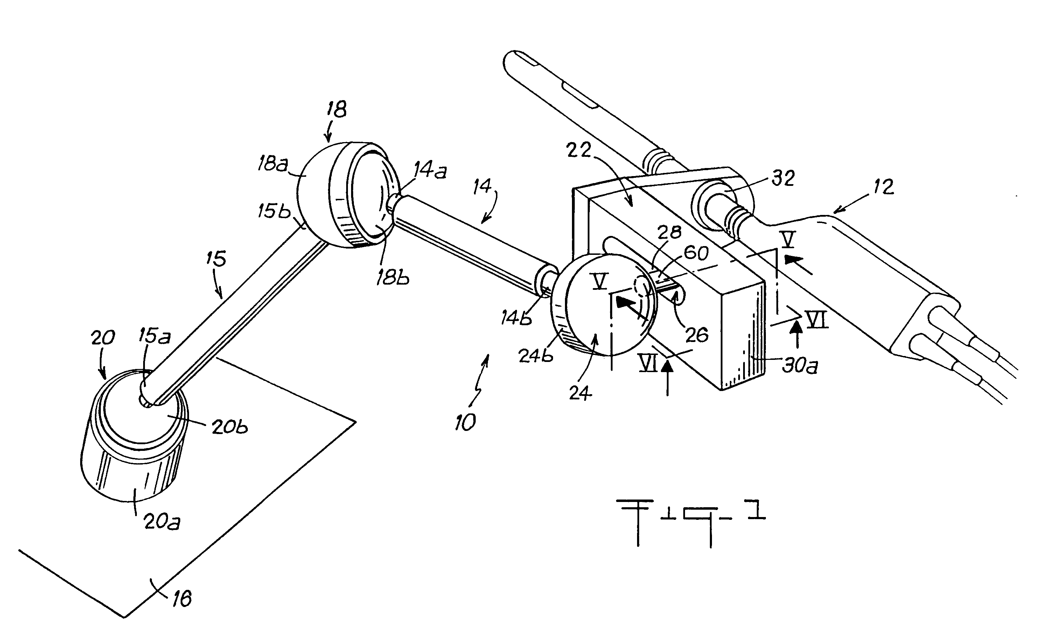

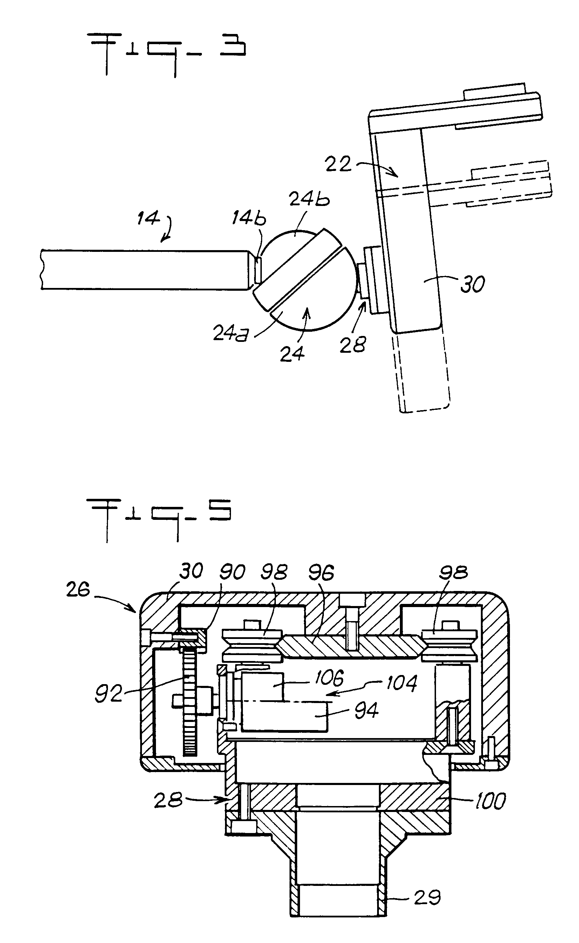

En référence plus particulièrement aux figures 1 à 3, on a représenté un dispositif de positionnement dans l'espace selon l'invention, représenté par le numéro de référence général 10 permettant le positionnement dans l'espace d'un instrument 12, par exemple une sonde endocavitaire ou une sonde d'exploration, en particulier de type échographique ou de type radiographique, en particulier par rayons X. Cette sonde est ici de type endorectal. Le dispositif de positionnement 10 comprend un bras 14 essentiellement rigide ayant une première extrémité 14a, et une deuxième extrémité 14b. La première extrémité 14a est articulée relativement à un bâti support 16 par au moins un axe d'articulation 18, 20, de préférence au moins deux axes d'articulation (18, 20). Par ailleurs, l'instrument 12 est monté sur un élément support 22 relié à la deuxième extrémité 14b du bras 14 par au moins un axe d'articulation 24.

Selon la présente invention, ce dispositif de positionnement est caractérisé en outre en ce que l'élément support 22 de l'instrument 12 comprend un dispositif de translation 26 qui est représenté en détail dans la coupe de la figure 5, permettant de réaliser une translation de l'instrument 12 par rapport à la deuxième extrémité 14b du bras 14.

Selon un mode de réalisation avantageux de l'invention, ce dispositif de translation 26 comprend un élément fixe 28 solidaire de l'articulation 24 de la deuxième extrémité 14b du bras 14 par un organe de liaison 29 et un élément mobile 30, déplaçable en translation relativement à l'élément fixe 28, l'élément mobile 30 supportant l'instrument 12 (voir figure 5).

L'élément mobile 30 peut être déplacé en translation relativement à l'élément fixe 28 par tout système mécanique bien connu à l'homme de l'art. Par exemple, il peut s'agir d'un système à crémaillère 90 engrenant avec une roue dentée 92 d'un moteur d'entraînement 94 monté sur une pièce support 100, par exemple en U faisant partie de l'élément fixe 28. Cette pièce support 100 supporte, par exemple, également des galets 98 disposés de part et d'autre d'un rail de guidage 96 solidaire de l'élément mobile 30, afin d'assurer un guidage précis du déplacement en translation de l'élément mobile 30.

Il peut également être prévu un moyen de mesure de translation 104, pour la mesure de la valeur de la translation de l'élément mobile 30. Ce moyen de mesure de translation peut, par exemple, comprendre un codeur de type potentiomètre 106 bien connu à l'homme de l'art.

Selon un mode de réalisation préféré de l'invention, l'élément mobile 30 comprend des moyens 32, que l'on voit mieux à la figure 6, de mise en rotation de l'instrument 12 représenté en trait fantôme.

Ces moyens de mise en rotation 32 peuvent, par exemple, comprendre un moteur d'entraînement en rotation 34 par un système de roue dentée 36 coopérant avec un élément d'entraînement 38 tel qu'une courroie crantée, entraînant en rotation une roue dentée 40 comportant un orifice central 42 coaxial à la roue 40 à l'intérieur duquel est inséré l'instrument 12, par exemple un instrument comprenant une sonde d'exploration, par visualisation, en particulier une sonde d'exploration, en particulier une sonde d'exploration échographique de type ultrasonique.

Les moyens de mise en rotation 32 peuvent comprendre un organe de débrayage permettant une mise en rotation manuelle de l'instrument 12. Ici, cet organe de débrayage est tout simplement constitué par le système d'entraînement par courroie crantée 38 combinée au moteur 34. On comprend en effet que lors d'une mise en rotation manuelle de la sonde 12, cette mise en rotation manuelle réalise un entraînement en rotation de la roue 40 supportant l'instrument 12, de la courroie 38 et de la roue d'entraînement 36 du moteur 34 de manière tout à fait libre.

Selon encore un autre mode de réalisation particulièrement avantageux du dispositif selon l'invention, celui-ci comprend un moyen de mesure de rotation 50, pour la mesure de la valeur du déplacement en rotation des moyens 32, en particulier la roue 40. Ce moyen de mesure 50 peut, par exemple, comprendre un potentiomètre 52 pourvu d'une roue dentée 54 engrenant également avec une roue dentée 36 a solidaire de la roue 36 du moteur 34, ce qui permet de calculer la valeur du déplacement en rotation de la roue 40, donc de l'instrument 12, à partir du nombre de tours de rotation de la roue 54, comme cela est bien connu à l'homme de l'art.

Selon un autre mode de réalisation particulièrement avantageux de l'invention, l'élément mobile 30 a une forme en L, comme clairement visible à la figure 1 et à la figure 6 dont une branche 30a se déplace en translation relativement à l'élément fixe 28 et l'autre branche 30b supporte l'instrument 12. Cette autre branche 30b, en raison de la forme en L, est sensiblement perpendiculaire à la branche 30a. Cette autre branche 30b comprend un orifice traversant 31 d'axe sensiblement perpendiculaire à cette autre branche 30b, donc sensiblement parallèle à la branche 30a, dans lequel sont montés de manière coaxiale la roue dentée 40 et l'instrument 12. On comprend ainsi que l'axe de rotation de l'instrument 12 est parallèle à la branche 30a, ce qui constitue une caractéristique structurelle avantageuse de l'invention.

Selon un autre mode de réalisation actuellement préféré du dispositif de positionnement selon l'invention, au moins certaines des articulations 18, 20, 24 sont de type à rotule. Ici, toutes les articulations 18, 20 et 24 sont de type à rotule.

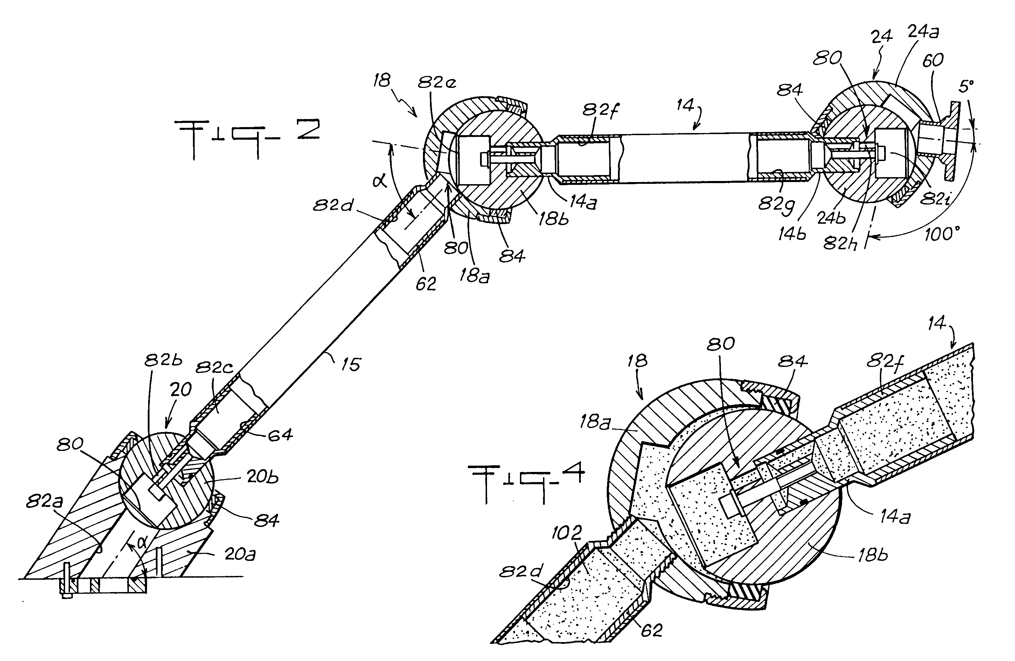

Selon un mode de réalisation particulier, tel que représenté, l'articulation 24 de la deuxième extrémité 14b du bras 14 est de type inverse par rapport à l'articulation 18 de la première extrémité 14a du bras. Dans le mode de réalisation préféré où les articulations 18, 20 et 24 sont de type rotule, ces articulations comprennent respectivement un élément fixe ou berceau, respectivement 18a, 20a, 24a, essentiellement sphérique tronqué, et un élément mobile essentiellement sphérique respectivement 18b, 20b et 24b, l'élément 24b étant clairement visible à la figure 2.

L'articulation 24 de la deuxième extrémité 14b du bras 14 étant inversée, l'élément dit fixe 24a supporte l'élément fixe 28 du dispositif de translation 22 auquel il est solidarisé par un élément de liaison 60.

Selon une autre caractéristique particulièrement avantageuse de l'invention, les articulations de type rotule 18, 20, 24 sont conçues de telle façon que l'organe de liaison tel que 60, 62, 64 de chaque élément fixe respectivement 24a, 28a, 20a forme un angle d'inclinaison aigu α par rapport à l'axe de symétrie de l'élément fixe. Cet angle d'inclinaison α peut être différent pour l'axe d'articulation 24 de la deuxième extrémité 14b du bras 14.

Avantageusement, cet angle d'inclinaison α est compris entre 20 et 65°, encore de préférence entre 35° et 65°. Un angle d'inclinaison particulièrement préféré est d'environ 55°, comme représenté.

Selon une autre caractéristique avantageuse de l'invention, il est prévu un deuxième bras 15, le dispositif de positionnement comprenant alors au total au minimum trois articulations 18, 20, 24, comme représenté. La première extrémité 15a du deuxième bras 15 peut être reliée à l'articulation 20 et la deuxième extrémité 15b à l'articulation 18 commune avec le premier bras 14.

Grâce à cette structure, on obtient un dispositif de positionnement particulièrement simple, permettant un maximum de degré de liberté et dont l'instrument 12 est monté déplaçable grâce au dispositif de déplacement 22.

Par ailleurs, il peut également être prévu des moyens 80 de blocage en position des articulations 18, 20, 24. Ces moyens de blocage 80 réalisent un blocage provisoire par déplacement relatif des éléments mobiles et fixes des articulations et sont en particulier de type pneumatique ou hydraulique.

Selon un mode de réalisation particulièrement avantageux, ces moyens de blocage 80 comprennent au moins un passage interne continu 82a à 82i, permettant le passage à l'intérieur du dispositif de positionnement 10 d'un fluide pneumatique ou hydraulique de mise sous pression, lors de l'activation du blocage, les bras 14 et 15 étant alors formés par des tubes. Le fluide pneumatique ou hydraulique est naturellement amené par des moyens d'amenée classiques (non représentés).

Ces moyens d'amenée réalisent de préférence une amenée du fluide pneumatique ou hydraulique sous pression 102, de préférence comprise entre 4 et 7 bar.

Il peut avantageusement être prévu vers l'extrémité libre de chaque élément fixe 18a, 20a, 24a de chaque articulation 18, 20, 24 un joint d'étanchéité 84, assurant simultanément un frottement entre les éléments fixes et mobiles des articulations.

Pour ce faire, le joint d'étanchéité 84 peut être réalisé en un matériau de nature différente de celui de l'élément mobile de l'articulation pour augmenter le frottement. Par exemple, ce joint d'étanchéité 84 est réalisé en PVC, tandis que l'élément mobile tel que 18a, 20a, 24b est réalisé en une matière plastique telle qu'un acétal, par exemple du polyoxyméthylène (POM).

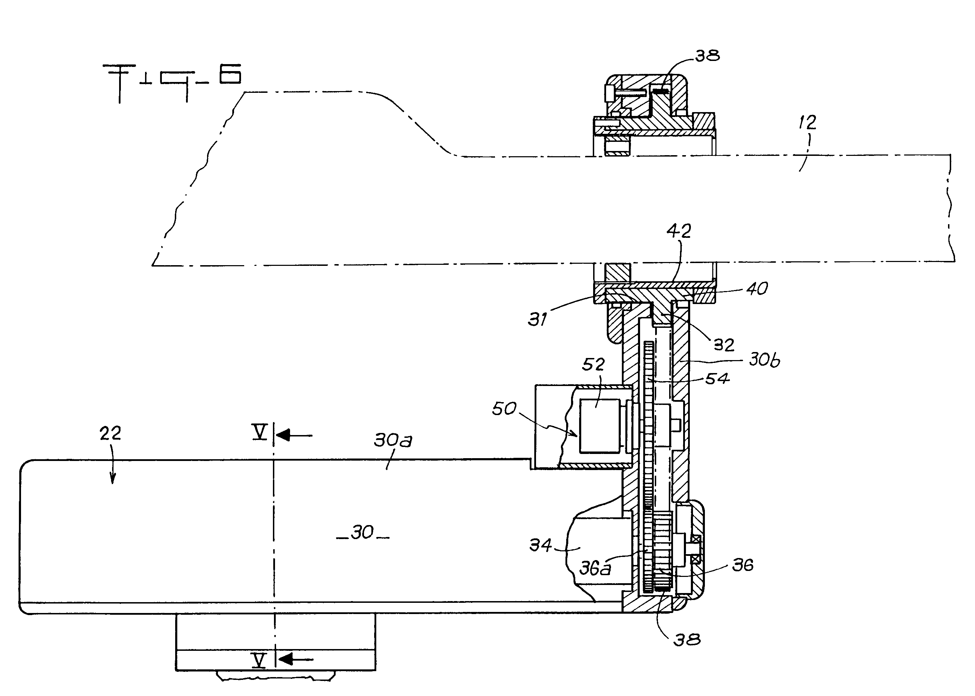

On conçoit ainsi que la structure complète, telle que représentée aux figures 1 à 6, fait partie intégrante de l'invention et fait donc partie intégrante de la présente description.

Cette structure permet de bloquer simultanément les rotules, ce qui est très pratique et assure un positionnement précis de l'instrument 12.

Toute structure nouvelle vis-à-vis de l'état de technique fait partie intégrante de l'invention.

L'invention comprend naturellement tous les moyens constituant des équivalents techniques des moyens décrits ainsi que leurs diverses combinaisons. Ceci s'applique en particulier au type de sonde utilisé.

## Revendications

1. Dispositif de positionnement dans l'espace d'un instrument (12), notamment comprenant une sonde d'exploration par visualisation, comprenant au moins un bras (14) essentiellement rigide ayant une première extrémité (14a) et une deuxième extrémité (14b), la première extrémité (14a) étant articulée relativement à un bâti support (16) par au moins un axe d'articulation (18, 20), et ledit instrument (12) étant monté sur un élément support (22) relié à la deuxième extrémité (14b) du bras (14) par au moins un axe d'articulation (24), caractérisé en ce que ledit instrument comprend une sonde endocavitaire ou une sonde d'exploration (12) notamment d'exploration par visualisation, en particulier une sonde échographique, de préférence de type rectal, permettant une exploration endorectale, et ledit élément support (22) de l'instrument (12) comprend un dispositif de translation (26) permettant de réaliser une translation de l'instrument comprenant ladite sonde endocavitaire ou d'exploration (12), par rapport à la deuxième extrémité (14b) du bras (14) en permettant de réaliser une opération, notamment une exploration, pour chacune des positions de translation.

2. Dispositif selon la revendication 1, caractérisé en ce qu'il comprend deux bras (14, 15) articulés entre eux par au moins une articulation, ainsi qu'à chacune de ses extrémités libres (15a, 14b respectivement), soit au total au moins trois articulations (18, 20, 24).

3. Dispositif selon la revendication 2, caractérisé en ce qu'il comprend des moyens (80) de blocage simultané des articulations (18, 20, 24).

4. Dispositif selon la revendication 3, dans lequel les articulations comprennent des éléments mobiles et des éléments fixes, caractérisé en ce que les moyens de blocage (80) réalisent un blocage provisoire par déplacement relatif des éléments mobiles et fixes des articulations et sont en particulier de type pneumatique ou hydraulique.

5. Dispositif selon l'une des revendications 3 ou 4, caractérisé en ce que les moyens de blocage (80) comprennent au moins un passage interne continu (82a, 82i), permettant le passage à l'intérieur du dispositif de positionnement (10) d'un fluide pneumatique ou hydraulique de mise sous pression, lors de l'activation du blocage, les bras (14, 15) étant alors formés par des tubes.

6. Dispositif selon la revendication 1, caractérisé en ce que le dispositif de translation (26) comprend un élément fixe (28) solidaire de l'articulation (24) de la deuxième extrémité (14b) du bras (14) par un organe de liaison (29) ; et un élément mobile (30), déplaçable en translation relativement à l'élément fixe (28), l'élément mobile (30) supportant l'instrument (12) ; ou inversement.

7. Dispositif selon la revendication 4 ou 6, caractérisé en ce que l'élément mobile (30) comprend des moyens (32) de mise en rotation de l'instrument (12), permettant un mouvement de rotation de l'instrument.

8. Dispositif selon la revendication 7, caractérisé en ce que les moyens de mise en rotation (32) précités comprennent un organe de débrayage permettant une mise en rotation manuelle de l'instrument (12).

9. Dispositif selon l'une des revendications 6 à 8, caractérisé en ce que l'élément mobile (30) a une forme en L, dont une branche (30a) se déplace en translation relativement à l'élément fixe (28) du dispositif de translation (26), et dont l'autre branche (30b) supporte l'instrument (12), avantageusement de manière rotative.

10. Dispositif selon l'une des revendications 1, 6 à 9, caractérisé en ce qu'au moins une articulation, de préférence toutes les articulations (18, 20, 24) sont des articulations de type rotule.

11. Dispositif selon la revendication 10, caractérisé en ce que l'articulation de type rotule (24) à la deuxième extrémité (14b) du bras (14) est de type inversé, de sorte que le bras (14) est solidarisé à sa première extrémité (14a) et à sa deuxième extrémité (14b) à la partie interne (24b), habituellement mobile, de l'articulation de type rotule (24).

12. Dispositif selon l'une des revendications 10 ou 11, caractérisé en ce qu'au moins certains des éléments fixes (18a, 20a, 24a) des articulations de type rotule (18, 20, 24) ont leur axe de symétrie formant un angle d'inclinaison aigu (α) relativement à l'axe général du bras (14) ; cet angle d'inclinaison (α) est avantageusement compris entre 20 et 65°, de préférence entre 35 et 60°, encore de préférence d'environ 55°.

13. Dispositif selon la revendication 12, caractérisé en ce que l'angle d'inclinaison (α) entre l'élément dit fixe (24a) de la dernière articulation (24) de type rotule à la deuxième extrémité (14b) du bras (14) et l'organe de liaison (29) à l'élément fixe (28) du dispositif de translation (22) est différent de celui des autres articulations de type rotule.

14. Dispositif selon l'une des revendications 1 à 13, caractérisé en ce qu'il comprend des moyens de mesure (50, 104) de déplacement de l'instrument (12) au moins en translation (104) et éventuellement en rotation (50).

15. Appareil de traitement thérapeutique, caractérisé en ce qu'il comprend un dispositif de positionnement (10) dans l'espace d'un instrument (12), comprenant une sonde endocavitaire ou une sonde d'exploration, tel que défini à l'une quelconque des revendications 1 à 14.

16. Appareil de traitement thérapeutique selon la revendication 15, caractérisé en ce que la sonde précitée est une sonde endorectale, permettant une exploration endorectale, avantageusement pour l'exploration de la prostate par voie endorectale, ladite sonde étant en particulier une sonde d'exploration par voie ultrasonique ou par voie radiographique, notamment par rayons X.

17. Appareil de traitement thérapeutique selon la revendication 15 ou 16, caractérisé en ce qu'il s'agit d'un appareil de traitement par hyperthermie, notamment de la prostate.

## Claims

1. A positioning device for positioning in space an instrument (12), notably comprising a display exploration probe, comprising at least one essentially rigid arm (14) having a first end (14a) and a second end (14b), the first end (14a) being hinged relative to a support frame (16) by at least one hinge axis (18, 20) and said instrument (12) being mounted on a support element (22) linked to the second end (14b) of said arm (14) through at least one hinge axis (24), characterized in that said instrument comprises an endocavitary probe or an exploration probe (12) notably for exploration through display, in particular an echographic probe, preferably of the rectal type, enabling an endorectal exploration, and said support element (22) of said instrument (12) comprises a translation device (26) enabling to perform a translation of said instrument comprising said endocavitary probe or said exploration probe (12), with regard to said second end (14b) of said arm (14) enabling to perform an operation, notably an exploration, for each one of the translation positions.

2. Device according to claim 1, characterized in that it comprises two arms (14, 15) hinged between them through at least one hinge, as well as at each one of its free ends (15a, 14b respectively), amounting in total to at least three hinges (18, 20, 24).

3. Device according to claim 2, characterized in that it comprises locking means (80) for simultaneously locking said hinges (18, 20, 24).

4. Device according to claim 3, wherein said hinges comprise moving parts and stationary parts, characterized in that said locking means (80) perform a provisional locking through a relative displacement of the moving and stationary parts of said hinges and are in particular of the pneumatic or of the hydraulic type.

5. Device according to one of claims 3 or 4, characterized in that said locking means (80) comprise at least one continuous internal path (82a, 82i), enabling the passage inside the positioning device (10) of a pneumatic or hydraulic fluid for accomodating under pressure, when activating the locking, said arms (14, 15) being then constituted by tubes.

6. Device according to claim 1, wherein said translation device (26) comprises a stationary part (28) secured to said hinge (24) of said second end (14b) of said arm (14) through a link member (29); and a moving part (30), displaceable in translation relatively to said stationary part (28), said moving part (30) supporting said instrument (12); or reversely.

7. Device according to claim 4 or 6, wherein said moving part (30) comprises rotating means (32) for rotating said instrument (12), to enable a rotation movement of said instrument.

8. Device according to claim 7, characterized in that said rotation means (32) comprise an unclutching member for enabling the instrument (12) to be rotated manually.

9. Device according to one of claims 6 to 8, characterized in that said moving part (30) is L-shaped, with one leg (30a) displaceable in translation relatively to said stationary part (28) of the translation device (26), and the other leg (30b) supports said instrument (12), advantageously rotatively.

10. Device according to one of claims 1, 6 to 9, characterized in that at least one hinge, preferably all the hinges (18, 20, 24) are of the ball-and-socket type joints.

11. Device according to claim 10, characterized in that said ball-and-socket type joint (24) at the second end (14b) of said arm (14) is of the reverse type so that said arm (14) is fixed to said first end (14a) and said second arm (14b) to the internal part (24b), usually moving, of said ball-and-socket type joint (24).

12. Device according to one of claims 10 or 11, characterized in that at least some of said stationary parts (18a, 20a, 24a) of said ball-and-socket type joints (18, 20, 24) have their symmetry axis forming an acute angle of inclination (α) relative to the general axis of said arm (14); said inclination angle (α) is advantageously ranging between 20 and 65°, and preferably between 35 and 60°, and more preferably of about 35°.

13. Device according to claim 12, characterized in that said angle of inclination (α) between said stationary part (24a) of the last hinge (24) of the ball-and-socket type joint at the second end (14b) of said arm (14) and the link member (29) to said stationary part (28) of said translation device (22) is different from that of the other ball-and-socket type joints.

14. Device according to one of claims 1 to 13, characterized in that it comprises measuring means (50, 104) for measuring the displacement of said instrument (12) at least in translation (104) and optionally in rotation (50).

15. A therapeutic treatment apparatus, characterized in that it comprises a positioning device (10) in space of an instrument (12), comprising an endocavitary probe or an exploration probe, such as defined in anyone of claims 1 to 14.

16. A therapeutic treatment apparatus according to claim 15, characterized in that said probe is an endorectal probe, enabling an endorectal exploration, advantageously for exploring the prostate endorectally, said probe being in particular an ultrasonic or radiographic, notably with X-rays, exploration probe.

17. A therapeutic treatment apparatus according to claim 15 or 16, characterized in that it is a treatment apparatus by hyperthermia, notably of the prostate.

## Patentansprüche

1. Vorrichtung zum Positionieren eines Instruments (12) mit insbesondere einer visuellen Untersuchungssonde im Raum, umfassend mindestens einen im wesentlichen starren Arm (14) mit einem ersten Ende (14a) und einem zweiten Ende (14b), wobei das erste Ende (14a) in bezug auf ein Traggestell (16) über mindestens eine Gelenkachse (18, 20) gelenkig befestigt ist und das Instrument (12) an einem Tragelement (22) montiert ist, welches über mindestens eine Gelenkachse (24) mit dem zweiten Ende (14b) des Arms (14) verbunden ist, dadurch gekennzeichnet, daß das Instrument eine Hohlraumsonde oder eine Untersuchungssonde (12), wie insbesondere eine visuelle Untersuchungssonde, im speziellen eine echografische Sonde, vorzugsweise vom Rektaltyp, zur Ermöglichung einer endorektalen Untersuchung, aufweist, und das Tragelement (22) für das Instrument (12) eine Translationseinrichtung (26) aufweist, mit der eine Translation des die Hohlraum- oder Untersuchungssonde (12) aufweisenden Instruments in bezug auf das zweite Ende (14b) des Arms (14) unter Ermöglichung einer Tätigkeit, insbesondere einer Untersuchung, bei jeder Translationsposition möglich ist.

2. Vorrichtung nach Anspruch 1, dadurch gekennzeichnet, daß sie zwei Arme (14, 15) aufweist, die mittels mindestens eines Gelenks aneinander sowie an jedem ihrer freien Enden (15a bzw. 14b) angelenkt sind, was insgesamt mindestens drei Gelenke (18, 20, 24) ergibt.

3. Vorrichtung nach Anspruch 2, dadurch gekennzeichnet, daß sie Blockiermittel (80) zum gleichzeitigen Blockieren der Gelenke (18, 20, 24) aufweist.

4. Vorrichtung nach Anspruch 3, bei welcher die Gelenke bewegliche Elemente und fixe Elemente aufweisen, dadurch gekennzeichnet, daß die Blockiermittel (80) durch Relativverschiebung der beweglichen und der fixen Elemente der Gelenke eine provisorische Blockierung herstellen und insbesondere vom pneumatischen oder hydraulischen Typ sind.

5. Vorrichtung nach einem der Ansprüche 3 oder 4, dadurch gekennzeichnet, daß die Blockiermittel (80) mindestens einen durchgehenden Innendurchlaß (82a, 82i) aufweisen, der den Durchtritt eines pneumatischen oder hydraulischen Druckmittels bei Aktivierung der Blockierung zum Inneren der Positioniervorrichtung (10) gestattet, wobei die Arme (14, 15) dann durch Rohre gebildet sind.

6. Vorrichtung nach Anspruch 1, dadurch gekennzeichnet, daß die Translationseinrichtung (26) ein durch ein Verbindungsorgan (29) mit dem Gelenk (24) des zweiten Endes (14b) des Arms (14) fest verbundenes fixes Element (28) und ein gegebenüber dem fixen Element (28) translatorisch verschiebbares bewegliches Element (30) aufweist, wobei das bewegliche Element (30) das Instrument (12) trägt; oder umgekehrt.

7. Vorrichtung nach Anspruch 4 oder 6, dadurch gekennzeichnet, daß das bewegliche Element (30) Mittel (32) zum Versetzen des Instruments (12) in Rotation aufweist, die eine Rotationsbewegung des Instruments gestatten.

8. Vorrichtung nach Anspruch 7, dadurch gekennzeichnet, daß die Mittel (32) zum Versetzen in Rotation ein Kupplungsorgan aufweisen, welches das händische Rotieren des Instruments (12) gestattet.

9. Vorrichtung nach einem der Ansprüche 6 bis 8, dadurch gekennzeichnet, daß das bewegliche Element (30) die Form eines L hat, dessen einer Schenkel (30a) in bezug auf das fixe Element (28) der Translationseinrichtung (26) translatorisch verschiebbar ist und dessen anderer Schenkel (30b) das Instrument (12), vorteilhafterweise drehbar, trägt.

10. Vorrichtung nach einem der Ansprüche 1, 6 bis 9, dadurch gekennzeichnet, daß mindestens ein Gelenk, vorzugsweise alle Gelenke (18, 20, 24), vom Kugelgelenktyp sind.

11. Vorrichtung nach Anspruch 10, dadurch gekennzeichnet, daß das Gelenk vom Kugelgelenktyp (24) am zweiten Ende (14b) des Arms (14) vom umgekehrten Typ ist, so daß der Arm (14) an seinem ersten Ende (14a) und an seinem zweiten Ende (14b) fest mit dem üblicherweise beweglichen Innenteil (24b) des Gelenks (24) vom Kugelgelenktyp verbunden ist.

12. Vorrichtung nach einem der Ansprüche 10 oder 11, dadurch gekennzeichnet, daß zumindest einige der fixen Elemente (18a, 20a, 24a) der Gelenke vom Kugelgelenktyp (18, 20, 24) mit ihrer Symmetrieachse einen spitzen Neigungswinkel (α) zur allgemeinen Achse des Arms (14) einschließen, welcher Neigungswinkel (α) vorteilhaft zwischen 20 und 65°, vorzugsweise zwischen 35 und 60°, noch bevorzugter etwa 55°, beträgt.

13. Vorrichtung nach Anspruch 12, dadurch gekennzeichnet, daß der Neigungswinkel (α) zwischen dem fixen Element (24a) des letzten Gelenks (24) vom Kugelgelenktyp am zweiten Ende (14b) des Arms (14) und dem Verbindungsorgan (29) am fixen Element (28) der Translationseinrichtung (22) unterschiedlich von jenem der anderen Gelenke vom Kugelgelenktyp ist.

14. Vorrichtung nach einem der Ansprüche 1 bis 13, dadurch gekennzeichnet, daß sie Mittel (50, 104) zum Messen von zumindest der translatorischen Verschiebebewegung (104) des Instruments (12) und gegebenenfalls der Rotationsbewegung (50) aufweist.

15. Apparat zur therapeutischen Behandlung, dadurch gekennzeichnet, daß er eine Vorrichtung (10) zum Positionieren eines Instruments (12) mit einer Hohlraumsonde oder einer Untersuchungssonde im Raum nach einem der Ansprüche 1 bis 14 aufweist.

16. Apparat zur therapeutischen Behandlung nach Anspruch 15, dadurch gekennzeichnet, daß die Sonde eine endorektale Sonde ist, die eine endorektale Untersuchung gestattet, vorteilhaft zur Untersuchung der Prostata auf endorektalem Weg, wobei die Sonde insbesondere eine Untersuchungssonde mittels Ultraschall oder mittels Radiografie, insbesondere mittels Röntgenstrahlen, ist.

17. Apparat zur therapeutischen Behandlung nach Anspruch 15 oder 16, dadurch gekennzeichnet, daß es sich um einen Apparat zur Behandlung mittels Hyperthermie, insbesondere der Prostata, handelt.
